# EUROPÄISCHE PATENTSCHRIFT

(11) **EP 1 343 557 B1**
(45) Veröffentlichungstag und Bekanntmachung des Hinweises auf die Patenterteilung: **22.09.2004**
(21) Anmeldenummer: 01985396.9
(22) Anmeldetag: 11.12.2001
(51) Int. Cl.: A61M 27/00

(54) **HYDROCEPHALUSVENTIL**
HYDROCEPHALUS VALVE
SOUPAPE POUR HYDROCEPHALE

(30) Priorität: 11.12.2000 DE 10061611; 03.02.2001 DE 10105315
(43) Veröffentlichungstag der Anmeldung: 17.09.2003
(73) Patentinhaber: Christoph Miethke Gmbh & Co. KG, 14532 Kleinmachnow (DE)
(72) Erfinder: MIETHKE, Christoph, 14558 Bergholz-Rehbrücke (DE)
(74) Vertreter: HOEGER, STELLRECHT & PARTNER Patentanwälte
(86) Internationale Anmeldenummer: PCT/EP2001/014585
(87) Internationale Veröffentlichungsnummer: WO 2002/047754

(56) Entgegenhaltungen:
- DE-A- 4 026 202
- DE-A- 19 654 990
- DE-A- 19 915 558
- FR-A- 2 772 278
- US-A- 5 980 480
- US-A- 6 022 333

## Beschreibung

Die Erfindung betrifft ein Hydrocephalusventil mit einem elektrisch betätigbaren Schaltventil, welches über eine als Zeitsteuerung ausgebildete Steuerung durch eine elektrische Betätigung zwischen einer Offenstellung und einer Schließstellung verstellbar ist, wobei das Schaltventil in der Offenstellung und in der Schließstellung bei nicht aktivierter elektrischer Betätigung stabil ist

Bei an Hydrocephalus erkrankten Menschen besteht das Problem, daß ein durch überschüssiges Hirnwasser entstehender erhöhter Hirninnendruck zu schwerwiegenden Problemen für die Betroffenen führt. So wird das Hirngewebe geschädigt und dauerhaft abgebaut, es kommt zu unterschiedlichen Symptomen wie Schwindel, Gangstörung, Kopfschmerzen, Übelkeit, Erbrechen und Demenz. Unbehandelt kann die Erkrankung letztendlich zum Tod des Patienten führen. Art und Umfang der auftretenden Beschwerden hängen ab von der Erkrankung zugrundeliegenden Ursachen, allgemeiner Konstitution, vor allem aber auch vom Alter des Patienten. Bei Säuglingen bewirkt der Druckanstieg ein unnatürliches Wachstum des Kopfes, bei Erwachsenen geht die Hirnsubstanz zugunsten des Wasseranteils im Schädelinneren schneller verloren.

Erst seit etwa 50 Jahren steht für Hydrocephaluskranke eine erfolgreiche Behandlungsmöglichkeit zur Verfügung. Dabei wird eine künstliche Drainage implantiert, die den Abfluß des Hirnwassern in andere Körperregionen ermöglicht, in denen die abgeleitete Flüssigkeit dann abgebaut werden kann. Die Steuerung des Abflusses wird dabei von Ventilen übernommen, die den erforderlichen Druck im Inneren des Kopfes sicherstellen sollen. Seitdem sind eine Vielzahl von verschiedenen technischen Lösungen vorgeschlagen worden, die die Behandlungsmöglichkeiten erweitern oder aber häufig auftretende Komplikationen verhindern oder einschränken sollen.

Bis heute haben sich drei verschiedene Ventilarten am Markt behaupten können: 1. Ventilsysteme mit einem einfachen, starren Differenzdruckventil, 2. Ventilsysteme mit einem postoperativ, perkutan verstellbaren Ventilsystem und 3. Hydrostatische Ventilsysteme. Das starre Differenzdruckventil der Gruppe 1 wird als Kugel-Konus-Konstruktion (US 5069663, DE 3020991), als Silikonschlitzventil oder als Membranventil angeboten. Die Ventile zeichnen sich dadurch aus, daß ihr Öffnungsverhalten auf die liegende Position des Patienten ausgerichtet ist. In der Stehendposition führen solche Ventile systematisch zu unphysiologisch niedrigem, negativem Druck im Kopf des Patienten, das kann zu schwerwiegenden Komplikationen führen.

Vertreter der zweiten Gruppe von Ventilen führen insofern eine Verbesserung ein, als diese Ventile zwar wie die der Gruppe 1 arbeiten, jedoch eine perkutane Verstellung der Öffnungscharakteristik erlauben, (US 4772257, EP 0 421 557 A2, US 5928182, EP 135 991 A1, GB 2143008 A, US 4551128,EP 0 060 369). Hierdurch wird eine individuelle Anpassung der Ventilfunktion an den einzelnen Patienten möglich. Allerdings beheben auch diese Ventile nicht die Schwierigkeit, daß die physikalischen Verhältnisse im Drainagesystem des Patienten sich in Abhängigkeit von der Haltung ändern. Sind die Ventile auf einen niedrigen Öffnungsdruck eingestellt, wird dies zwar einerseits das klinische Ergebnis begünstigen, andererseits wird gleichzeitig die Gefahr der Überdrainage in der Stehendposition dramatisch erhöht. Umgekehrt kann zwar die Einstellung auf einen sehr hohen Wert die Gefahr der Überdrainage reduzieren, gleichzeitig wird dadurch das zu erzielende klinische Ergebnis nachhaltig negativ beeinflußt, da der jetzt anliegende Öffnungsdruck für die Liegendposition deutlich zu hoch ist.
Abhilfe schaffen hier Ventile der Gruppe drei. Hydrostatische Ventile zeichnen sich dadurch aus, daß sie die sich ändernden physikalischen Bedingungen im Drainagesystem des Patienten berücksichtigen mit dem Ziel, die oben beschriebenen Probleme infolge einer Überdrainage zu vermeiden. Drei unterschiedliche Prinzipien werden hierbei genutzt.

Die älteste Konstruktion wurde im sogenannten Antisiphon-Device realisiert. Nach dem gleichen Prinzip sind bis heute mehrere unterschiedliche Konstruktionen am Markt angeboten worden (EP 0 670 740 B1, US 5800376, DE 2752087). Hierbei wird die Wirkung des negativen Drukkes am Auslaß des Ventils systematisch auf ein Minimum reduziert. Diesem Vorteil steht allerdings der gravierende Nachteil gegenüber, daß der Subcutandruck um das Ventilgehäuse einen erheblichen Einfluß auf die Arbeitsweise des Ventils nimmt. Durch Gewebswachstum oder ungünstige Patientenlage kann dieser Druck um erhebliche Werte variieren und dadurch sogar zum absoluten Ventilverschluß führen. Auch diese Ventile haben sich im Vergleich mit konventionellen Ventilen als nicht überlegen erwiesen (Drake, Toronto).

Das gleiche gilt für das zweite Prinzip der dritten Gruppe, dem Prinzip der sogenannten Flußkontrolle. Bei den flußregulierenden Ventilen soll sichergestellt werden, daß die abfließende Menge unabhängig vom am Ventil anliegenden Differenzdruck konstant gehalten wird. Während bei konventionellen Ventilen die Abflußmenge proportional zum anliegenden Differenzdruck steigt, wird dies bei flußregulierenden Ventilen verhindert (Siphonguard [Codman], Orbis Sigma Valve [Cordis], Diamond Valve [Phoenix] ; EP 798 012 A1, US 4627832, US 4776838). Im Mittel beträgt die natürliche Liquorproduktion 23 ml/h. Flußregulierende Systeme haben konkret folgende Probleme. 1. Es ist technisch unmöglich, den Wert für die erlaubte Abflußrate sicherzustellen. Varianzen im Rahmen des Produktionsprozesses bleiben zu groß (Aschoff, Schoener). 2. Die natürliche Varianz der Produktion bleibt systematisch unberücksichtigt. Liegen die individuellen Werte zu hoch oder zu niedrig, kann dies sowohl zur Überdrainage als auch zur Unterdrainage führen. 3. Die Flußregulierung wird gesteuert über extrem kleine Querschnitte am Öffnungsmechanismus. Partikel im Liquor wie etwa zelluläre Bestandteile nehmen dramatisch Einfluß auf die Funktion und können das Ventil sehr leicht verstopfen. Internationale Vergleichsstudien haben gezeigt, daß dieses Prinzip die Behandlungsergebnisse des Hydrocephalus nicht verbessern konnte (Drake et al).

Im Gegensatz dazu wurden nach Einführung von gravitationsunterstützten Ventilen signifikante Verbesserungen festgestellt (Meier, Sprung, Kiefer). Zwei verschiedene technische Lösungen werden am Markt angeboten. Der erste Ansatz realisiert die Flußsteuerung durch die schwerkraftgesteuerte Umschaltung von zwei parallel angeordneten Ventilen (DE 4401422, DE 4307387, EP614673). Diese Konstruktion stellt also zwei unterschiedliche Drucksituationen im Ventrikelsystem des Patienten in Abhängigkeit von seiner Haltung ein. Beim zweiten Ansatz wird die Gewichtskraft von Kugeln ausgenutzt, um einen lageabhängig veränderlichen Öffnungsdruck einzustellen (EP 0 617 975,

EP 0 115 973, DE 19535637). Obwohl durch solche Ventilsysteme viele Probleme gelöst werden konnten, bleiben auch hier folgende Aspekte ungelöst:
1. Eine Anpassung der Ventilcharakteristik an wachstums- oder altersbedingte Veränderungen oder anderweitige Änderungen von physiologischen Randbedingungen ist nicht möglich.
2. Eine gezielte nicht-invasive Verstellung der Ventileigenschaften mit unterschiedlichen Einstellungen für unterschiedliche Körperlagen des Patienten ist nicht möglich.
3. Eine konsequente Therapierung von Patienten bis hin zur Abstellung der vielleicht überflüssig gewordenen Drainage ist nicht möglich.
4. Die angemessene Anpassung der Liquordrainage an individuelle Besonderheiten ist nicht möglich.
5. Ventile bleiben eine Engstelle der Ableitung. Die Erhöhung der Abflußsicherheit durch Bereitstellung von breiteren Öffnungskanälen auch im Ventilsitz wäre wünschenswert.
6. Alle bisher angebotenen Lösungen basieren ausschließlich auf dem Differenzdruckprinzip. Andere Parameter, die ebenfalls Einfluß auf die sinnvolle Steuerung bei der Liquordrainage haben könnten, werden nicht erfaßt. Denkbar wäre beispielsweise die Einbeziehung von Muskelpotentialen oder anderen elektronischen Signalen. Solche Signale können bei den bisher angebotenen Lösungen nicht berücksichtigt werden.
7. Die intelligente, situationsabhängig wertende Steuerung von Ventileigenschaften ist bei bisherigen Lösungen unmöglich.
8. Die nachträgliche Analyse von Vorfällen ist nicht möglich. Oft bleibt die Erklärung der Ursachen für Zwischenfälle bei Vermutungen.

Teilweise können diese Probleme durch Lösungen überwunden werden, wie sie in der DE 19915558 A1 beschrieben sind. Dort wird ein elektronisch geregeltes Implantat vorgeschlagen, das den Hirndruck von Hydrocephalus-Patienten regelt. Allerdings ist die Erfassung der Druckwerte im Patienten mit großen Unsicherheiten verbunden, und daher ist dieses Verfahren nicht geeignet, um die Flüssigkeitsabfuhr auf Dauer befriedigend zu regeln.

In der DE 19654990 A1 ist ein ähnliches System beschrieben, bei dem die Abflußmenge durch das Drainagesystem über elektromagnetische Stellglieder geregelt wird, beispielsweise werden die Charakteristika eines Federventils verstellt, die Größe eines Spaltes in der Ableitung, und ähnliches. Dies macht eine außerordentlich komplizierte Konstruktion des Ventiles notwendig, außerdem muß man dabei davon ausgehen, daß die Regelgrößen, also Fluß und Druck, über die Zeit konstant sind und nur gegebenenfalls im Rahmen von Arztbesuchen anzupassen sind, um dann wieder für einen längeren Zeitraum Gültigkeit zu haben.

In der FR-A-2772278 ist eine externe Drainagevorrichtung für Hirnflüssigkeit beschrieben, bei der der Druck im Gehirn als Steuergröße für die Regulierung des Abflusses verwendet wird. Diese Regulierung kann über eine Pumpe oder verschiedene Ventilkonstruktionen erfolgen, beispielsweise ein Ventil mit einem elektromagnetisch verschiebbaren Ventilkörper, der eine flexible Katheterleitung zusammendrückt, um den Durchfluß durch diese Katheterleitung zu unterbrechen.

Im Rahmen der DE 40 26 202 A1 wird ein Hydrocephalusventil beschrieben, welches einen elektromagnetisch verschiebbaren ZweiWege-Ventilkörper umfaß.

Der Erfindung liegt die Aufgabe zugrunde, bei einem gattungsgemäßen Hydrocephalusventil einen Aufbau zu realisieren, der eine sichere und einfache Funktion gewährleistet und gleichzeitig einen besonders einfachen Aufbau aufweist.

Diese Aufgabe wird bei einem Hydrocephalusventil der eingangs beschriebenen Art erfindungsgemäß dadurch gelöst, daß das Schaltventil einen kugelförmigen Ventilkörper umfaßt, der in der Schließstellung auf einer Ventilöffnung dichtend aufliegt und in der Offenstellung seitlich neben der Ventilöffnung in einer Vertiefung ruht.

Die Betätigung kann dabei einen Schieber umfassen, der den Ventilkörper einerseits und die Ventilöffnung sowie die Vertiefung andererseits relativ zueinander verschiebt. Die Verschiebung kann dabei eine Verschiebung des Ventilkörpers sein, es ist aber auch möglich, daß die Ventilöffnung mit der daneben angeordneten Vertiefung relativ zu einem feststehenden Ventilkörper verschoben wird.

Die Betätigung kann einen elektromagnetischen Antrieb, einen piezoelektrischen Antrieb oder ein stromdurchflossenes Bimetall umfassen, hier sind auch noch weitere an sich geläufige Antriebe möglich.

Die Zeitsteuerung hält das Hydrocephalusventil über einen bestimmten Zeitraum offen und schließt es dann über einen bestimmten Zeitraum, beispielsweise kann das Ventil nachts während sicherer Liegephasen langzeitig geöffnet bleiben, während tagsüber der Schalter nur einmal pro Stunde für 30 Sekunden oder einige Minuten geöffnet wird.

Besonders vorteilhaft ist es, wenn die Zeitsteuerung programmierbar ist, so daß man die Öffnungsdauern und die Schließungsdauern durch ein solches Programm verändern kann. Auf diese Weise ist es beispielsweise möglich, für jeden Patienten ein eigenes Zeitsteuerungsprogramm zu entwickeln, das auf seine Bedürfnisse zugeschnitten ist. Es ist auch möglich, für ein und denselben Patienten mehrere derartige Zeitprogramme vorzusehen, so daß beispielsweise in der Schlafphase ein anderes Zeitsteuerprogramm verwendet wird als bei der Wach- und Stehphase. Insbesondere ist es für den Arzt auf diese Weise möglich, durch eine entsprechende Programmierung das Abflußverhalten der Flüssigkeit jederzeit zu verändern und den Bedürfnissen anzupassen.

Dabei kann es sich um eine reine Steuerung handeln, der Zeitablauf wird also durch das Programm unmittelbar vorgegeben und bleibt unbeeinflußt durch die dadurch erzielten Resultate.

Bei einer anderen bevorzugten Ausführungsform kann jedoch vorgesehen sein, daß die Zeitsteuerung durch Signale von mindestens einem Meßinstrument beeinflußbar ist.

Bei diesen Meßinstrumenten kann es sich beispielsweise um ein Druckmeßgerät handeln, das Druckwerte im Gehirn, in der Umgebung oder an sonst interessierenden Stellen mißt, einen Lagesensor, der feststellt, ob der Patient liegt, steht oder sich bewegt, ein Meßinstrument zur Bestimmung von Muskelpotentialen oder Hirnstrompotentialen etc.

Die Beeinflussung führt dabei zu einer Änderung des Zeitsteuerungsprogrammes, so daß Abänderungen bei den Offenzeiten und bei den Schließzeiten eintreten.

Günstig ist es, wenn der Zeitsteuerung ein Datenspeicher zugeordnet ist, der alle Öffnungs- und Schließzustände über der Zeit erfaßt und damit eine Dokumentation bildet, die jederzeit eine Überprüfung ermöglicht.

Bei dem beschriebenen Hydrocephalusventil wird nicht das Öffnungsverhalten, beispielsweise die Schließkraft eines Ventilkörpers oder die Größe eines Durchflußquerschnittes, beeinflußt, sondern es wird lediglich bei unverändertem Durchflußquerschnitt zwischen Offenstellung und Schließstellung umgeschaltet, so daß sichergestellt ist, daß auch durch Gewebeteile in der Flüssigkeit und andere Einlagerungen keine Störung des Abflußverhaltens bei geöffnetem Hydrocephalusventil auftritt.

Zusätzlich kann gemäß einer weiteren bevorzugten Ausführungsform vorgesehen sein, daß die Zeitsteuerung das Öffnungsverhalten des Schließventils verändert. Das Öffnungsverhalten kann beispielsweise durch Querschnittsveränderung, durch Veränderung von Federkräften etc. geändert werden.

Die nachfolgende Beschreibung bevorzugter Ausführungsformen der Erfindung dient im Zusammenhang mit der Zeichnung der näheren Erläuterung. Es zeigen:
- Figur 1:: ein Blockdiagramm einer einfachen Zeitsteuerung für ein elektromagnetisches Schaltventil;
- Figur 2:: eine Ansicht ähnlich Figur 1 bei einer Zeitsteuerung, die über die Signale eines Lagesensors beeinflußbar ist;
- Figur 3:: eine Ansicht ähnlich Figur 2 mit einer Zeitsteuerung, die über Signale von mehreren Meßgeräten beeinflußbar ist;
- Figur 4:: eine schematische Längsschnittansicht eines bistabilen Schaltventils und
- Figur 5:: ein typisches Steuerprofil für eine Zeitsteuerung.

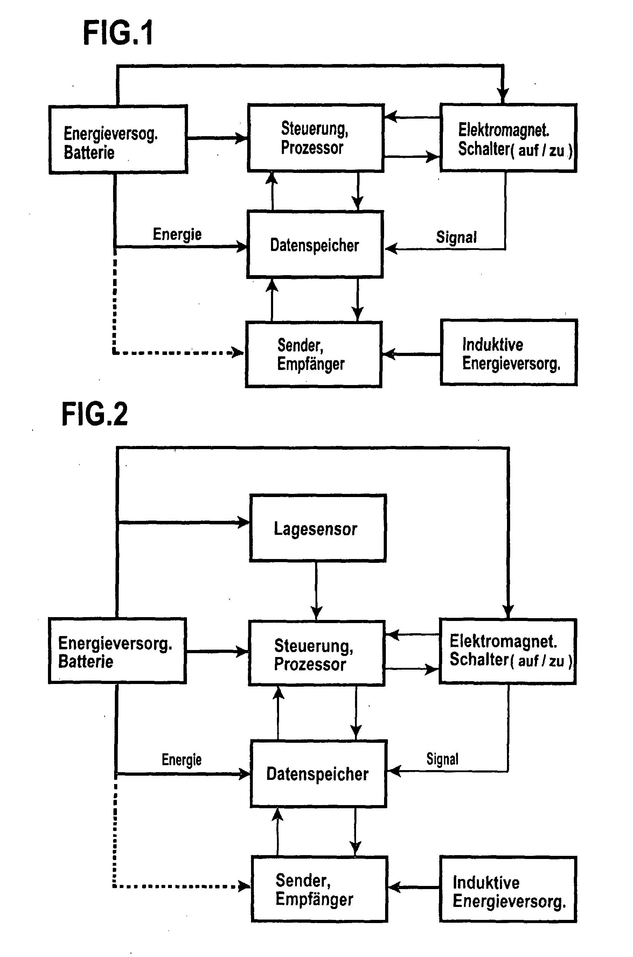

Das in der Zeichnung dargestellte Hydrocephalusventilsystem umfaßt eine Energieeinheit (Batterie), einen Prozessor, einen Datenspeicher, einen elektromagnetischen Schalter, eine Sende- und Empfangseinheit sowie optional eine Einheit zur induktiven Energieeinspeisung. Figur 1 zeigt das Blockdiagramm für die Wirkungsweise der Erfindung.

In Figur 1 ist die einfachste Bauweise eines Ventilsystems dargestellt. Über den Sender wird ein zeitabhängiges Steuersignal s(t) in den Datenspeicher eingespielt. Das Steuersignal s(t) enthält eine für jeden Patienten individuell erstellbare Steuerinformation über den Öffnungszustand des elektromagnetischen Schalters zu jedem Zeitpunkt t. So kann beispielsweise der Öffnungszustand nachts länger andauern als tagsüber. Es besteht die Möglichkeit, ein individuelles Profil entsprechend den Lebensgewohnheiten oder Bedürfnissen des Patienten zu erstellen. Dieses Öffnungsprofil läßt sich etwaigen Änderungen jederzeit anpassen.
Der Datenspeicher gibt die Information an die Steuereinheit weiter, die wiederum die elektromechanische Komponente steuert. Der Schalter gibt die Information über seinen Zustand (offen oder geschlossen) an den Prozessor und den Datenspeicher weiter. Die Sende- und Empfangseinheit kann vorzugsweise über eine induktive Energieversorgung aktiviert und mit Energie versorgt werden. Die Energieversorgung kann aber auch über die Batterie erfolgen.

Eine aufwendigere Variante der Erfindung ist in Figur 2 dargestellt. Hier wird die in Figur 1 dargestellte Einheit durch einen Lagesensor ergänzt. Dieser Sensor gibt die Haltung des Patienten an die Steuereinheit weiter, die gemäß der gesendeten Programmierung den Sollwert der Ventilbetätigung ändert.

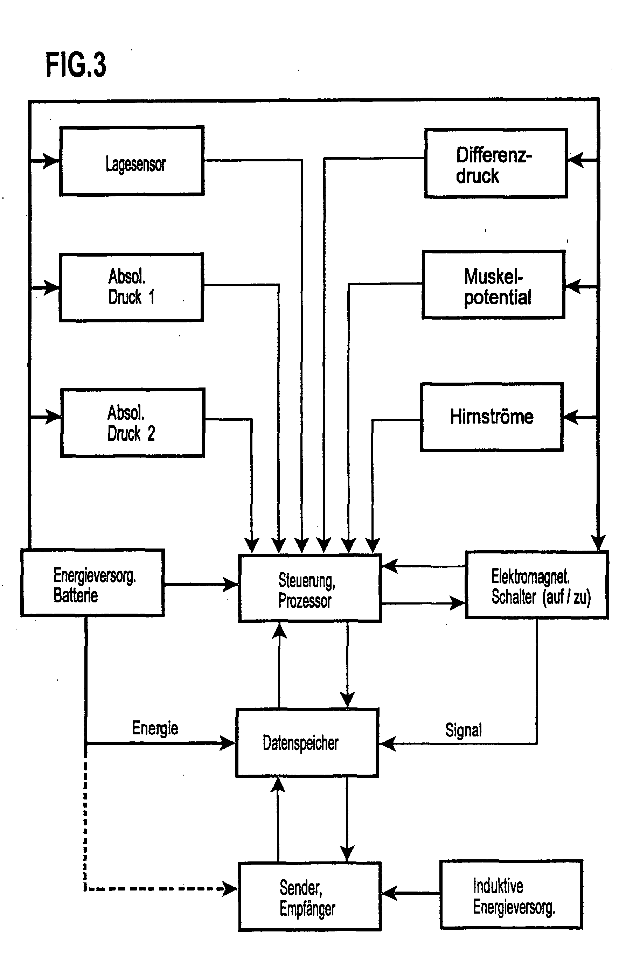

Neben dem Lagesensor können auch weitere Kennwerte bestimmende Meßeinrichtungen in die Sollwertbestimmung einbezogen werden. Hierzu können Muskelpotentiale genauso wie abgenommene Druckwerte bestimmt werden. Vor allem die Einbeziehung und intelligente Auswertung von gemessenen Druckwerten kann zur Optimierung der Drainage herangezogen werden. Die Erfassung der Druckwerte erfolgt allerdings nicht, um wie bei konventionellen Drainagen eine vom Differenzdruck am Ventil gesteuerte Drainage zu etablieren. Der Druck kann aber dazu genutzt werden, kurzfristige Änderungen zu erfassen und entsprechend zu berücksichtigen. Das Problem der Drift von Druckaufnehmern wird dadurch unbedeutend. So könnte beispielsweise die Erfassung des Absolutdruckes vor und hinter dem Schalter sichere Anhaltspunkte für die aktuelle Drainagesituation liefern. Steht beispielsweise bei geschlossenem Schalter ein Patient auf, führt dies sowohl oberhalb als auch unterhalb des Schalters zu charakteristischen Zuständen, die entsprechend kontrolliert werden können. Die Auswertung solcher Zustände kann extrakorporal erfolgen nach Aussenden der aufgenommenen Daten und zur Erstellung von verbesserten Regelalgorithmen genutzt werden. Auch die ausschließliche Erfassung des Differenzdruckes am Schalter bietet Erkenntnisse zur Optimierung des Regelalgorithmus. Figur 3 zeigt ein Blockschaltbild für eine solche aufwendige Ausführung der Erfindung.

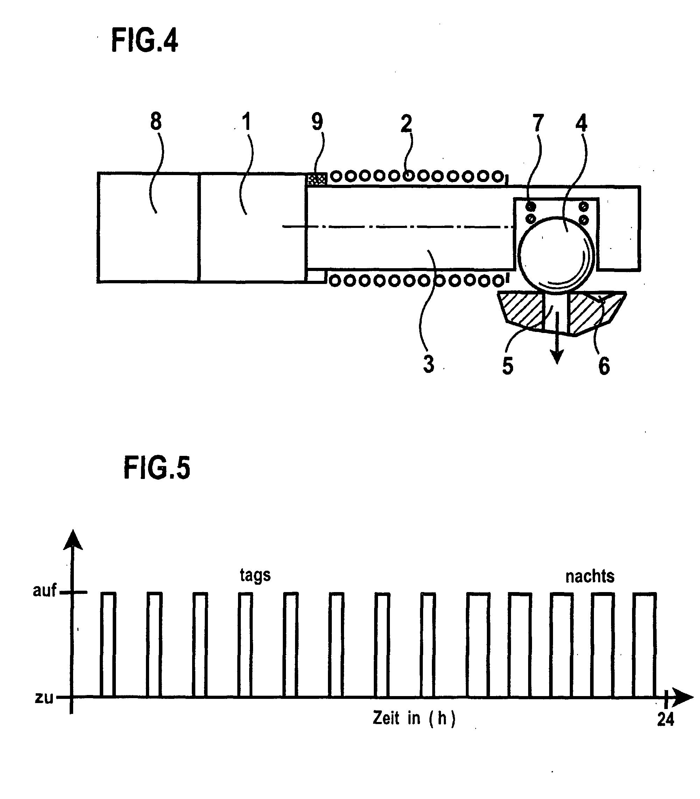

Figur 4 zeigt ein Ausführungsbeispiel für einen elektromechanischen Schalter zum Öffnen und Schließen der Drainage. Eine Elektronik 1 wird durch eine Batterie 8 mit Strom versorgt. Je nach Schaltrichtung wird an einer Spule 2 eine Spannung angelegt, wodurch ein Magnetfeld erzeugt wird, das einen Schieber 3 bewegt. Der Schieber 3 kann zwei verschiedene Ruhezustände einnehmen, die durch eine Feder 7 gesichert werden. Die Auswahl der Feder erfolgt in der Art, daß einerseits die Feder den Sitz einer Kugel 4 auch bei Erschütterungen sichert, andererseits aber die Kraft zur Verschiebung der Kugel 4 gering bleibt. Entweder ruht sie in der Öffnung in einem Ventilauslaß 5 (Position 1) oder aber in einer Sackbohrung 6 (Position 2). Die Auslaßbohrung am Ventilauslaß 5 weist typischerweise einen Durchmesser von etwa 1 mm auf, dies entspricht dem Innendurchmesser einer typischen Drainageleitung. Wird die Kugel 4, die vorzugsweise aus einem harten und leichten Material hergestellt ist, beispielsweise Aluminiumoxidkeramik, und die vorzugsweise einen etwa 3-fach größeren Durchmesser hat als die Bohrung des Ventilauslasses 5, in die Position 1 geschoben, ist die Drainage verschlossen. Wird die Kugel 4 in Position 2 geschoben, ist der gesamte Querschnitt der Bohrung freigegeben. Diese Maximalöffnung minimiert die Verstopfungsgefahr am Schalter. Über einen Detektor 9 kann die Lage des Schiebers 3 fortlaufend erfaßt werden. Die Ansteuerung des Schiebers 3 kann sowohl als reine Zeitsteuerung erfolgen gemäß einem vorgegebenen individuell patientenabhängigen Zeitprofil oder aber auch durch einen aufwendigeren Algorithmus (gemäß Blockschaltbild 2 und 3) berechnet werden.

Insbesondere die Einbeziehung der Patientenlage kann den Regelalgorithmus optimieren helfen.

Figur 5 zeigt ein Beispiel für eine denkbare Steuervorgabe. Die Vorgaben für die Schalterstellung können je nach Patient und Tageszeit variieren. Beispielsweise kann nachts während sicherer Liegephasen das Ventil langzeitig geöffnet bleiben, während tagsüber der Schalter möglicherweise nur einmal pro Stunde für 30 Sekunden oder einige Minuten geöffnet wird. Das Signal des Lagesensors kann die Öffnungszeit verkürzen oder verlängern. Das Steuersignal wird durch die Haut an das Implantat gesendet. Die Erfassung von Zustandsdaten im Implantat können bei Bedarf abgerufen werden.
Andere Ausführungsbeispiele für einen solchen Schalter können als bewegte Membran realisiert werden. Die Erzeugung der Bewegung kann über bewegte Leiter im Magnetfeld ebenso erfolgen wie über die Kraft, die auf Magnete im Magnetfeld ausgeübt wird, das zur Verstellung angelegt wird. Weitere Möglichkeiten der Verstellung bestehen in der piezoelektrischen Bewegungserzeugung oder durch die Ansteuerung von Bimetallen.

## Patentansprüche

1. Hydrocephalusventil mit einem elektrisch betätigbaren Schaltventil, welches über eine als Zeitsteuerung ausgebildete Steuerung durch eine elektrische Betätigung zwischen einer Offenstellung und einer Schließstellung verstellbar ist, wobei das Schaltventil in der Offenstellung und in der Schließstellung bei nicht aktivierter elektrischer Betätigung stabil ist, **dadurch gekennzeichnet, daß** das Schaltventil einen kugelförmigen Ventilkörper (4) umfaßt, der in der Schließstellung auf einer Ventilöffnung (5) dichtend aufliegt und in der Offenstellung seitlich neben der Ventilöffnung (5) in einer Vertiefung (6) ruht.

2. Ventil nach Anspruch 1, **dadurch gekennzeichnet, daß** die Betätigung einen Schieber (3) umfaßt, der den Ventilkörper (4) einerseits und die Ventilöffnung (5) sowie die Vertiefung (6) andererseits relativ zueinander verschiebt.

3. Ventil nach einem der Ansprüche 1 oder 2, **dadurch gekennzeichnet, daß** die Betätigung einen elektromagnetischen Antrieb umfaßt.

4. Ventil nach einem der Ansprüche 1 oder 2, **dadurch gekennzeichnet, daß** die Betätigung einen piezoelektrischen Antrieb umfaßt.

5. Ventil nach einem der Ansprüche 1 oder 2, **dadurch gekennzeichnet, daß** die Betätigung ein stromdurchflossenes Bimetall umfaßt.

6. Ventil nach einem der voranstehenden Ansprüche, **dadurch gekennzeichnet, daß** die Zeitsteuerung programmierbar ist.

7. Ventil nach einem der voranstehenden Ansprüche, **dadurch gekennzeichnet, daß** die Zeitsteuerung durch Signale von mindestens einem Meßinstrument beeinflußbar ist.

8. Ventil nach Anspruch 7, **dadurch gekennzeichnet, daß** das Meßinstrument ein Druckmeßgerät ist.

9. Ventil nach einem der Ansprüche 7 oder 8, **dadurch gekennzeichnet, daß** das Meßinstrument ein Lagesensor ist.

10. Ventil nach einem der Ansprüche 7 bis 9, **dadurch gekennzeichnet, daß** das Meßinstrument Muskelpotentiale bestimmt.

11. Ventil nach einem der Ansprüche 7 bis 10, **dadurch gekennzeichnet, daß** das Meßinstrument Hirnstrompotentiale bestimmt.

12. Ventil nach einem der voranstehenden Ansprüche, **dadurch gekennzeichnet, daß** der Zeitsteuerung ein Datenspeicher zugeordnet ist.

13. Ventil nach einem der voranstehenden Ansprüche, **dadurch gekennzeichnet, daß** die Zeitsteuerung das Öffnungsverhalten eines Schließventiles verändert.

## Claims

1. A hydrocephalus valve comprising an electrically actuatable switching valve which is movable between an open position and a closed position by an electrical actuation means by a controller formed as a time controller, wherein the switching valve is stable in the open position and in the closed position when the electrical actuation means is non-activated, **characterised in that** the switching valve comprises a spherical valve body (4) which is supported on a valve opening (5) so as to form a seal in the closed position and rests in a recess (6) laterally of the valve opening (5) in the open position.

2. A valve according to claim 1, **characterised in that** the actuation means comprises a slider (3) which effects relative displacement of the valve body (4) on the one hand and the valve opening (5) and the recess (6) on the other hand.

3. A valve according to either one of claims 1 and 2, **characterised in that** the actuation means comprises an electromagnetic drive.

4. A valve according to either one of claims 1 and 2, **characterised in that** the actuation means comprises a piezoelectric drive.

5. A valve according to either one of claims 1 and 2, **characterised in that** the actuation means comprises a current-carrying bimetal.

6. A valve according to any one of the preceding claims, **characterised in that** the time controller is programmable.

7. A valve according to any one of the preceding claims, **characterised in that** the time controller can be influenced by signals from at least one measuring instrument.

8. A valve according to claim 7, **characterised in that** the measuring instrument is a pressure meter.

9. A valve according to either one of claims 7 and 8, **characterised in that** the measuring instrument is a position sensor.

10. A valve according to any one of clams 7 to 9, **characterised in that** the measuring instrument determines muscular potentials.

11. A valve according to any one of claims 7 to 10, **characterised in that** the measuring instrument determines brain flow potentials.

12. A valve according to any one of the preceding claims, **characterised in that** a data store is associated with the timer.

13. A valve according to any one of the preceding claims, **characterised in that** the time controller changes the opening behaviour of a closed valve.

## Revendications

1. Soupape pour hydrocéphale comportant une soupape de commutation pouvant être actionnée électriquement, qui est réglable au moyen d'une unité de commande agencée sous la forme d'une unité de commande temporelle, au moyen d'un dispositif d'actionnement électrique, entre une position ouverte et une position fermée, la soupape de commutation étant stable dans la position ouverte et dans la position fermée dans le cas où le dispositif d'actionnement électrique n'est pas activé, **caractérisée en ce que** la soupape de commutation comprend un corps de soupape (4) de forme sphérique, qui, dans la position fermée, s'applique de manière étanche sur une ouverture de soupape (5) et, dans la position ouverte, est logée latéralement à côté de l'ouverture de soupape (5) dans un renfoncement (6).

2. Soupape selon la revendication 1, **caractérisée en ce que** le dispositif d'actionnement comprend un poussoir (3), qui déplace l'un par rapport à l'autre d'une part le corps de soupape (4) et d'autre part l'ouverture de soupape (5) ainsi que le renfoncement (6).

3. Soupape selon l'une des revendications 1 ou 2, **caractérisée en ce que** le dispositif d'actionnement comporte un dispositif d'entraînement électromagnétique.

4. Soupape selon l'une des revendications 1 ou 2, **caractérisée en ce que** le dispositif d'actionnement comprend un dispositif d'entraînement piézoélectrique.

5. Soupape selon l'une des revendications 1 ou 2, **caractérisée en ce que** le dispositif d'actionnement comprend un élément bimétallique traversée par le courant.

6. Soupape selon l'une des revendications précédentes, **caractérisée en ce que** l'unité de commande temporelle est programmable.

7. Soupape selon l'une des revendications précédentes, **caractérisée en ce que** l'unité de commande temporelle peut être influencée par des signaux d'au moins un instrument de mesure.

8. Soupape selon la revendication 7, **caractérisée en ce que** l'instrument de mesure est un appareil de mesure de pression.

9. Soupape selon l'une des revendications 7 ou 8, **caractérisée en ce que** l'instrument de mesure est un capteur de position.

10. Soupape selon l'une des revendications 7 à 9, **caractérisée en ce que** l'instrument de mesure détermine des potentiels musculaires.

11. Soupape selon l'une des revendications 7 à 10, **caractérisée en ce que** l'instrument de mesure détermine des potentiels électriques du cerveau.

12. Soupape selon l'une des revendications précédentes, **caractérisée en ce qu'**une mémoire de données est associée à une unité de commande temporelle.

13. Soupape selon l'une des revendications précédentes, **caractérisée en ce que** l'unité de commande temporelle modifie le comportement d'ouverture d'une soupape de fermeture.
